# EUROPEAN PATENT APPLICATION

(11) **EP 1 216 990 A1**
(43) Date of publication of application: **26.06.2002**
(21) Application number: 00128048.6
(22) Date of filing: 21.12.2000
(51) Int. Cl.: C07C 403/24

(54) **Process for the preparation of carotenoids**

(71) Applicant: AVENTIS ANIMAL NUTRITION S.A., 92164 Antony Cedex (FR)
(72) Inventor: Quesnel, Yannick, 69006 Lyon (FR); Flacher, Richard, 69009 Lyon (FR); Casati, Jean-Pierre, 69520 Grigny (FR)
(74) Representative: Mérigeault, Shona

(57) **Abstract**

A process for the production of carotenoids which comprises reacting a retinal with a titanium(IV) compound in the presence of zinc and an inert solvent, in particular a process for the production of beta-carotene from retinal.

## Description

The present invention relates to a process for the production of carotenoid from retinal and in particular, to a process for the production of carotenoid by reacting retinal in the presence of a titanium compound wherein titanium is in the (IV) oxidation state.

Carotenoids, in particular beta-carotene, can be synthesised from retinals. In general, the synthesis involves reacting a titanium (II) compound with retinal in the presence of a reducing agent. In particular, US Patent No. 4225734 discloses a process for the production of beta- carotene by reacting a titanium (II) species with retinal in the presence of a reducing agent such as lithium aluminium hydride or zinc. This process requires an excess of reducing agent and the reaction must be carried out under reaction conditions which requires refluxing the solvent. The final product is contaminated with chlorinated compounds.

We have now found that carotenoids can be prepared in a simple manner and avoids the problem of contamination.

Accordingly, the present invention provides a process for the production of carotenoids which comprises reacting a retinal with a titanium(IV) compound in the presence of zinc and an inert solvent.

The process of the present invention provides the advantage over the prior art processes in that the final product is not contaminated

The process of the present invention provides a method of producing carotenoids from retinals. Retinals suitable for use in the present invention include retinal, hydroxy retinal or protected forms thereof such as ester protection, silyl protection, and ether protection, THP protection; oxy retinal or protected forms thereof such as ketals. The preferred retinals are retinal, 4-oxo retinal, 4-hydroxy retinal, 3-hydroxy-4-oxo-retinal and 3-hydroxy-retinal. The corresponding carotenoids are prepared according to the reaction scheme:

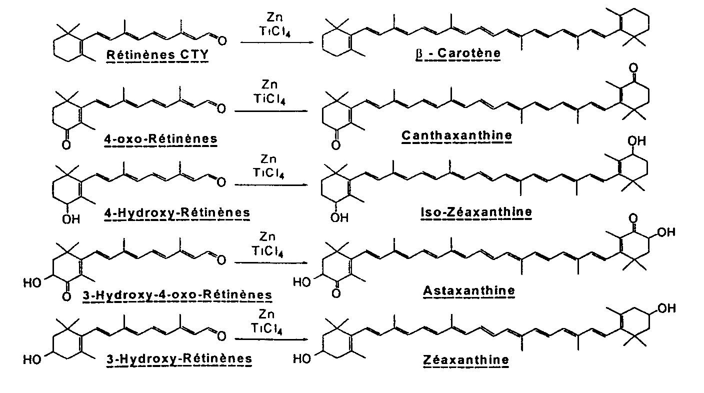

The process of the present invention involves the reaction between a titanium (IV) compound and retinal. Suitably, the titanium compound is a halide, for example a chloride or bromide, especially titanium tetrachloride. Suitably, the ratio of the titanium compound to retinal is from 0.1 to 3 equivalents, preferably from 0.5 to 1.5 equivalents.

The process of the present invention further requires the presence of zinc. Suitably, zinc is present as zinc metal and may be used in the form of powder, granules, fines and chips. The amount of retinal to zinc is suitably at least 1 equivalents of zinc, preferably from 1 to 5, especially from 1 to 1.3 equivalents.

The process of the present invention is carried out in the presence of an inert solvent. Solvents suitable for use in the process of the present invention include solvents such as dimethylformamide (DMF), dimethylsulphoxide (DMSO); ethers such as ^{*tert*}-butyl methyl ether (MTBE), tetra hydrofuran (THF), di-isopropyl ether; nitriles such as acetonitrile; esters such as ethyl acetate and isopropyl acetate and chlorocompounds such as chlorobenzene. It is preferred to use an ester or a chlorinated compound, especially an ester such as ethyl acetate. The amount of solvent used in the process of the present invention is suitable from 5 to 30 g of solvent per gram of retinal, preferably from 10 to 15 g of solvent per gram of retinal.

The process may be carried out at a temperature of from 0°C up to the reflux temperature of the solvent, preferably from 18 to 25°C. The process may be carried out under atmospheric or elevated pressure, preferably the process is carried out under atmospheric pressure. Operating under such mild conditions is a particular advantage of the process of the present invention as it prevents the amount of by-products.

The present invention will now be illustrated with reference to the following examples:

### Example 1: Production of Beta-Carotene in ethyl acetate

0.645 ml of titanium tetrachloride (0.6 equivalents, 5.87 mmole) was added drop by drop to 0.802 mg of zinc powder (1.25 equivalents, 11.74 mmol) in a suspension of 20 ml of ethyl acetate. The resulting mixture was stirred for 30 minutes. 3g of retinal (91%, 9.718 mmol) in a solution of 12 ml of ethyl acetate was then added over a 10 minutes period. A further 3 ml of ethyl acetate was introduced through the entrance nozzle to wash residual zinc and the resulting mixture left stirring for 1 hour at ambient, temperature. The mixture was then hydrolysed with 25 ml of hydrochloric acid (1N) and the mixture stirred for a further 30 minutes. The resulting two phases were then decanted and the organic phase washed with 25 ml of water. The ethyl acetate phase containing the beta-carotene product was dried and evaporated under vacuum.

Analysis of the resulting product using high performance liquid chromatography (HPLC) and NMR indicated only beta-carotene. Conversion of the retinal was 100% and the amount of product obtained was 2.61g with 100% selectivity.

### Example 2: Production of Beta-Carotene in ^{tert}-butyl methyl ether

The procedure of Example 1 was repeated but using ^{*tert*}-butyl methyl ether as the solvent. 0.822 ml of titanium tetrachloride (2.7 equivalents, 7.5 mmole) was added drop by drop to 0.631 mg of zinc powder (3.47 equivalents, 9.65 mmol) in a suspension of 15 ml of ^{*tert*}-butyl methyl ether. The resulting mixture was stirred for 30 minutes. 0.789 g of retinal (2.78 mmol) in a solution of 5 ml of ^{*tert*}-butyl methyl ether was then added over a 10 minutes period. A further 3 ml of ^{*tert*}-butyl methyl ether was introduced and the resulting mixture left stirring for 1.5 hour at ambient, temperature. The mixture was then hydrolysed with 25 ml of hydrochloric acid(1N) and the mixture stirred for a further 30 minutes. The content of the flask was then decanted and the organic phase washed with 25 ml of water. ^{*tert*}-butyl methyl ether phase containing the beta-carotene product was dried and evaporated under vacuum.

Analysis of the resulting product using HPLC and NMR indicated beta-carotene. Conversion of the retinal was 100% and the amount of product obtained was 0.693 g with 93% yield.

### Example 3: Production of Beta-Carotene in acetonitrile

The procedure of Example 1 was repeated but using acetonitrile as the solvent. 0.822 ml of titanium tetrachloride (2.7 equivalents, 7.5 mmole) was added drop by drop to 0.631 mg of zinc powder (3.47 equivalents, 9.65 mmol) in a suspension of 15 ml of refluxing acetonitrile. The resulting mixture was stirred for 30 minutes. 0.789 g of retinal (2.78 mmol) in a solution of 5 ml of acetonitrile was then added over a 10 minutes period. A further 3 ml of acetonitrile was introduced and the resulting mixture left stirring for 1.5 hour at reflux. The mixture was then hydrolysed with 25 ml of hydrochloric acid (1N) and the mixture stirred for a further 30 minutes. The crude product was then extracted with dichloromethane, the organic layer containing the beta-carotene product was dried and evaporated under vacuum.

Analysis of the resulting product using HPLC and NMR indicated beta-carotene and retinal. Conversion was 100% and the amount of product obtained was 0.708 g with 95% yield.

### Example 4: Production of Beta-Carotene in dimethylformamide (DMF)

The procedure of Example 1 was repeated but using dimethylformamide as the solvent. 0.464 ml of titanium tetrachloride (0.6 equivalents, 4.22 mmole) was added drop by drop to 0.575 mg of zinc powder (1.25 equivalents, 8.79 mmol) in a suspension of 15 ml of DMF at ambiant temperature. The resulting mixture was stirred for 30 minutes. 2 g of retinal (7.03 mmol) in a solution of 5 ml of DMF was then added over a 10 minutes period. A further 5 ml of DMF was introduced to rinced the dropping funnel and the resulting mixture left stirring for 5 hours at ambiant temperature. The mixture was then hydrolysed with 25 ml of hydrochloric acid (1N) and the mixture stirred for a further 30 minutes. The crude product was then extracted with ethyl acetate, the organic layer containing the beta-carotene product was dried and evaporated under vacuum. The crude product was purified by flash chromatography on silica gel yielding 0.912 g of beta - carotene (Yield 48 %).

## Claims

1. A process for the production of carotenoids which comprises reacting a retinal with titanium(IV) compound in the presence of zinc and an inert solvent.

2. A process as claimed in claim 1 in which the retinal is selected from retinal, hydroxy retinal or protected forms thereof; oxy retinal or protected forms thereof

3. A process as claimed in claim 2 in which the retinal is selected from retinal, 4-oxo retinal, 4-hydroxy retinal, 3-hydroxy-4-oxo-retinal and 3-hydroxy-retinal.

4. A process as claimed in any one of the preceding claims in which the titanium compound is titanium tetrachloride.

5. A process as claimed in claim 4 in which the titanium compound is present in an amount of titanium to retinal of from 0.1 to 3 equivalents.

6. A process as claimed in any one of the preceding claims in which zinc is present in an amount of retinal to zinc of at least 1 equivalent

7. A process as claimed in any one of the preceding claims in which the solvent is a an ether, an ester, a chlorocompound, dimethylformamide or dimethylsulphoxide.

8. A process as claimed in claim 5 in which the solvent is an ester, preferably ethyl acetate.

9. A process as claimed in any one of the preceding claims in which the solvent is present in an amount of from 5 to 30 g of solvent per gram of retinal.

10. A process as claimed in any one of the preceding claims for the production of beta-carotene from retinal.

11. A process as claimed in any one of the preceding claims carried out at a temperature of from 18 to 25°C.
